# FASCICULE DE BREVET EUROPEEN

(11) **EP 3 445 493 B1**
(45) Date de publication et mention de la délivrance du brevet: **17.02.2021**
(21) Numéro de dépôt: 17742477.7
(22) Date de dépôt: 19.06.2017
(51) Int. Cl.: B01L 3/00, B01L 9/00, B01J 19/10, B01L 9/06, B06B 3/00

(54) **DISPOSITIF DE SEGMENTATION D'ECHANTILLONS D'ADN**
VORRICHTUNG ZUR DNA-PROBENFRAGMENTIERUNG
DEVICE FOR DNA SAMPLE FRAGMENTATION

(30) Priorité: 19.04.2016 FR 1653466
(43) Date de publication de la demande: 27.02.2019
(73) Titulaire: Etablissement Français du Sang, 93210 Saint Denis (FR)
(72) Inventeur: MAHDJOUBI, Cyrus, 56530 Queven (FR); ALIZADEH, Mehdi, 35000 Rennes (FR)
(74) Mandataire: Novagraaf Technologies
(86) Numéro de dépôt international: PCT/FR2017/051609
(87) Numéro de publication internationale: WO 2017/182763

(56) Documents cités:
- EP-A1- 2 511 380
- US-A- 2 657 668
- US-A1- 2002 009 015
- US-A1- 2012 238 736

## Description

### 1. Domaine de l'invention

La présente divulgation concerne le domaine de la segmentation de l'ADN qui consiste à obtenir, à partir d'un brin d'ADN comportant un grand nombre de bases nucléotidiques, plusieurs portions de brin comportant un nombre de bases nucléotidiques plus restreint. La présente divulgation concerne plus particulièrement un dispositif de segmentation d'échantillons d'ADN.

### 2. Art antérieur

Il est connu des dispositifs de segmentation d'échantillons d'ADN, les échantillons étant mis en solution dans un contenant dédié. De tels dispositifs comportent classiquement une cuve destinée à recevoir un liquide, la cuve étant équipée d'un moyen de génération d'ondes ultrasonores pour propager des ondes ultrasonores dans le liquide ; et un élément support reposant sur la cuve destiné à recevoir le contenant. Le moyen de génération d'ondes ultrasonores est conçu pour générer un champ d'ondes ultrasonores avec des propriétés prédéfinies dans un espace restreint de la cuve. Cette caractéristique limite le nombre d'échantillons d'ADN qu'il est possible de segmenter simultanément dans le dispositif. Un processus de segmentation dure en moyenne une minute. Actuellement, le nombre maximum d'échantillons segmentés simultanément par un tel dispositif est de l'ordre de 8. Or, les échantillons sont classiquement stockés dans des plaquettes comportant une centaine de puits pour pouvoir contenir une centaine d'échantillons. Dans une perspective de productivité, il serait intéressant de segmenter simultanément tous les échantillons d'une plaquette.

En outre, les dispositifs de segmentation d'échantillons d'ADN imposent souvent l'utilisation de contenants spécifiques, en particulier lorsque le contenant joue un rôle dans la génération ou la transmission d'ondes ultrasoniques. Ceci impose un transvasement des échantillons d'un récipient d'origine vers un contenant spécifique, ce qui constitue une manipulation chronophage. Par ailleurs, ces récipients spécifiques représentent un coût supplémentaire pour l'utilisateur.

### 3. Objectifs de l'invention

L'invention propose une solution visant à pallier les inconvénients précités tout en garantissant un dispositif au moins aussi performant que l'existant avec un coût de production restreint, en particulier la durée du processus de segmentation est maintenu à environ une minute et l'homogénéité de la longueur des brins d'ADN obtenus suite à la segmentation est équivalente à l'existant. L'invention propose une solution résolvant le problème technique de la création d'un champ ultrasonore sensiblement homogène dans un volume contenant un grand nombre d'échantillons d'ADN sans utilisation de contenant spécifique. Un objectif de l'invention est de permettre l'utilisation de contenants standards dans le dispositif de segmentation d'échantillons d'ADN et notamment l'utilisation de plaquettes capables de contenir une centaine d'échantillons, voire plus, dans le but d'accroître la productivité d'un tel dispositif.

### 4. Résumé de l'invention

La présente divulgation concerne un dispositif de segmentation d'échantillons d'ADN mis en solution dans un contenant, le dispositif comportant une cuve destinée à recevoir un liquide, la cuve étant équipée d'un moyen de génération d'ondes ultrasonores pour propager des ondes ultrasonores dans le liquide ; et un premier élément support reposant sur la cuve, le dispositif étant caractérisé en ce qu'il comporte en outre un deuxième élément support comportant un orifice traversant destiné à recevoir le contenant, le deuxième élément support étant suspendu audit premier élément support à une certaine distance dudit premier élément support par au moins un élément de suspension formant au moins une liaison rotule de sorte à permettre l'immersion d'une portion inférieure du contenant dans le liquide. On entend par liaison rotule une liaison liant complètement deux pièces en translation mais les laissant libres en rotation. Une telle liaison comporte donc 3 degrés de liaisons en translation et 3 degrés de liberté en rotation. L'élément de suspension formant liaison rotule permet d'atténuer substantiellement la transmission d'ondes ultrasonores parasites du premier élément support vers le deuxième élément support grâce à un effet amortisseur de la liaison rotule. Plus généralement, le dispositif est conçu pour inhiber au maximum la transmission d'ondes ultrasonores parasites au contenant. L'élimination des ondes ultrasonores parasites contribue à créer un champ d'ondes ultrasonores sensiblement homogène au voisinage du contenant. En outre, le contenant n'est pas imposé par le dispositif et des formes variées de contenant conviennent. Selon un mode de réalisation avantageux, le contenant est une plaquette comprenant une pluralité de puits. On utilise indifféremment le terme plaque ou plaquette. De préférence, cette plaquette est une plaquette standard communément employée pour transporter et/ou traiter des échantillons d'ADN.

Selon un mode de réalisation particulier, l'élément de suspension est déformable afin de limiter la propagation des ondes parasites entre le premier élément support et le deuxième élément support.

Selon un mode de réalisation particulier, l'élément de suspension comporte un moyen de réglage agencé pour modifier la distance entre le premier élément support et le deuxième élément support. Le moyen de réglage permet de régler l'horizontalité du deuxième élément support et donc du contenant et de régler la position du deuxième élément support par rapport au fond de la cuve et donc la position du contenant par rapport au liquide.

Selon un mode de réalisation particulier, le moyen de génération d'ondes ultrasonores comporte au moins un transducteur ultrasonore transformant un signal électrique en un signal mécanique. De préférence, le moyen de génération d'ondes ultrasonores comporte une pluralité de transducteurs disposés selon une grille afin de créer un champ d'ondes ultrasonores relativement homogène au voisinage du contenant.

Selon un mode de réalisation particulier, le moyen de génération d'ondes ultrasonores comporte en outre un générateur de signal agencé pour alimenter en signal électrique le transducteur.

Selon un mode de réalisation particulier, le générateur de signal comporte un module de génération de signaux périodiques. Par exemple, le générateur de signal comporte au moins un oscillateur piézoélectrique.

Selon un mode de réalisation particulier, le générateur de signal comporte en outre un amplificateur de signal permettant d'amplifier le signal généré par le module de génération de signaux périodiques avant de transmettre le signal au transducteur.

Selon un mode de réalisation particulier, le générateur de signal comporte en outre un module de balayage en fréquence et/ou en phase de sorte à générer au moins deux signaux ayant des fréquences et/ou des phases distinctes dans une bande de fréquences prédéterminée. Le balayage en fréquence et/ou en phase permet d'éviter l'apparition de ventres et de nœuds fixes en régime stationnaire en présence d'ondes périodiques.

Selon un mode de réalisation particulier, le moyen de génération d'ondes ultrasonores est fixé sur une face inférieure externe de la cuve. Par exemple, les transducteurs sont collés sur le fond de la cuve au moyen d'une colle adaptée à la transmission des ondes ultrasonores ou sont soudés à la cuve. Le moyen de génération d'ondes ultrasonores transmet les ondes ultrasonores à la face inférieure de la cuve, puis la face inférieure de la cuve transmet à son tour les ondes ultrasonores au liquide.

Selon un mode de réalisation particulier, lorsque le dispositif est en position de fonctionnement, le moyen de génération d'ondes ultrasonores est placé sensiblement à la verticale de l'orifice du second élément support de sorte à être centré par rapport à l'orifice. La verticale est définie comme étant la direction de la pesanteur. La présence du moyen de génération d'ondes ultrasonores à la verticale de l'orifice du second élément support et donc à la verticale du contenant contribue à créer un champ d'ondes ultrasonores homogène au voisinage du contenant par effet de symétrie. Toutefois, la symétrie est optionnelle.

Selon un mode de réalisation particulier, le dispositif comporte un deuxième moyen de génération d'ondes ultrasonores pour réduire, voire annuler, les ondes ultrasonores parasites de manière active.

Selon un mode de réalisation particulier, le premier élément support comporte un orifice traversant de sorte à permettre à un utilisateur d'accéder à l'orifice du deuxième élément support. L'accès à l'orifice du deuxième élément support permet de placer un contenant dans le deuxième élément support ou de l'en retirer.

Selon un mode de réalisation particulier, l'élément de suspension comporte au moins trois chaînes en liaison rotule avec la plaque supérieure et en liaison rotule avec le support. Les trois chaînes sont réparties de sorte à permettre de maintenir le deuxième élément support dans une position horizontale tout en garantissant les degrés de mobilité d'une liaison rotule. L'horizontalité est définie comme étant perpendiculaire à la direction verticale.

Selon un mode de réalisation particulier, le deuxième élément support est distant des faces latérales de la cuve d'une distance prédéterminée, par exemple une distance de 10 centimètres, de sorte à limiter l'exposition du contenant aux effets de bord au voisinage des faces latérales créés par la réflexion des ondes ultrasonores par les faces latérales. Cette caractéristique contribue à l'homogénéité du champ d'ondes ultrasonores au voisinage du contenant.

Selon un mode de réalisation particulier, le dispositif comporte en outre un châssis extérieur dans lequel est placée la cuve. Le châssis permet de supporter la cuve et d'isoler l'environnement extérieur des ondes ultrasonores se propageant dans la cuve.

Selon un mode de réalisation particulier, le dispositif comporte un couvercle. Le couvercle permet d'isoler le contenu de la cuve de l'environnement extérieur dans un souci de sécurité et d'hygiène.

Selon un mode de réalisation particulier, le dispositif comporte en outre un dispositif de trop-plein destiné à assurer la présence d'un niveau prédéterminé de liquide dans la cuve. Il est nécessaire que le niveau du liquide soit suffisamment haut pour immerger la portion du contenant comprenant les échantillons d'ADN à segmenter. Cependant, il est préférable que le deuxième élément support ne soit pas immergé afin d'éviter les effets de bord liés à la réflexion des ondes ultrasonores sur le deuxième élément support, ce qui nuirait à l'homogénéité du champ d'ondes ultrasonores.

Selon un mode de réalisation particulier, le dispositif de trop-plein comprend un orifice de vidange afin d'évacuer une portion excédentaire du liquide.

Selon un mode de réalisation particulier, le dispositif comporte en outre un moyen de réglage de l'horizontalité de la cuve afin de garantir une immersion homogène de tous les puits du contenant dans le liquide.

Selon un mode de réalisation particulier, le moyen de réglage de l'horizontalité de la cuve comporte des pieds réglables.

Selon un mode de réalisation particulier, le moyen de réglage de l'horizontalité de la cuve comporte un niveau à bulle d'air.

### 5. Liste des figures

D'autres caractéristiques et avantages innovants ressortiront de la description ci-après, fournie à titre indicatif et nullement limitatif, en référence aux dessins annexés, dans lesquels :
- Les figures la et 1b représentent une vue schématique et en coupe d'un dispositif respectivement selon un premier et un deuxième mode de réalisation de l'invention ;

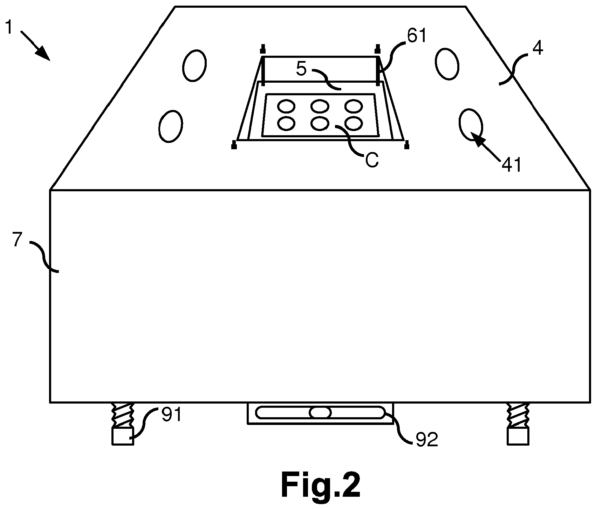

- La figure 2 représente une vue schématique en perspective du dispositif de la figure la ou de la figure 1b ; et
- La figure 3 représente un schéma fonctionnel d'un moyen de génération d'ondes ultrasonores selon l'invention.

### 6. Description détaillée

Aux figures la, 1b et 2 est représenté un dispositif 1 de segmentation d'échantillons d'ADN. Les échantillons d'ADN A à segmenter sont mis en solution dans une solution S et placés dans un contenant C. Par exemple, le dispositif 1 de segmentation permet d'obtenir à partir d'un génome comportant sensiblement 3 milliards de bases nucléotidiques, plusieurs portions de brin comportant sensiblement 400 bases. La solution S utilisée est typiquement de l'eau ou un gel aqueux. Le contenant C est préférentiellement un contenant standard, par exemple une plaquette comportant une pluralité de puits disposés selon un quadrillage, ou une rangée de tubes à essais.

Le dispositif 1 comporte une cuve 2, un moyen de génération d'ondes ultrasonores 3, un premier élément support 4, un deuxième élément support 5, un élément de suspension 6, un châssis 7, un couvercle 8 et un moyen de réglage de l'horizontalité 9 de la cuve.

La cuve 2 est un récipient étanche conçu pour recevoir un liquide L, typiquement de l'eau. La cuve 2 est avantageusement métallique et plus particulièrement en acier inoxydable de sorte à éviter l'oxydation de la cuve 2 au contact de l'eau et à faciliter son nettoyage dans un souci d'hygiène. La cuve 2 a par exemple une forme parallélépipédique et comporte une face inférieure et quatre faces latérales. D'un point de vue dimensionnel et à titre purement illustratif, la face inférieure est sensiblement de forme rectangulaire et ses côtés mesurent entre 25 et 35 centimètres. Les faces latérales ont une dimension verticale d'une dizaine de centimètres, la verticale étant définie par la direction de la pesanteur.

La cuve 2 est placée dans le châssis 7. Le châssis est un récipient capable d'isoler l'environnement extérieur des ondes ultrasonores se propageant dans la cuve 2. Le châssis 7 est avantageusement métallique et plus particulièrement en acier inoxydable. Le châssis 7 a par exemple une forme parallélépipédique et comporte une face inférieure et quatre faces latérales. D'un point de vue dimensionnel et à titre purement illustratif, la face inférieure est sensiblement de forme rectangulaire et ses côtés mesurent environ une quarantaine de centimètres. Les faces latérales ont une dimension verticale d'une vingtaine de centimètres.

Les faces latérales de la cuve 2 comportent des bords supérieurs reposant sur les faces latérales du châssis 7 de sorte qu'un espace soit présent entre la face inférieure externe de la cuve 2 et la face inférieure interne du châssis 7. Avantageusement, les interfaces entre les bords supérieurs de la cuve 2 et les faces latérales du châssis 7 comportent des joints 10 limitant la propagation d'ondes parasites entre la cuve 2 et le châssis 7.

Le premier élément support 4 a par exemple sensiblement une forme de plaque comportant des rebords. D'un point de vue dimensionnel et à titre purement illustratif, la plaque est sensiblement de forme rectangulaire et ses côtés mesurent entre 25 et 35 centimètres. Le premier élément support 4 s'emboîte de manière amovible sur les rebords des faces latérales de la cuve de sorte à garantir une position sensiblement fixe du premier élément support 4 par rapport à la cuve 2. Avantageusement, l'emboîtement du premier élément support 4 avec la cuve présente un jeu par exemple comblé par des joints amortisseurs 10 limitant la propagation d'ondes parasites entre la cuve 2 et le premier élément support 4. Le premier élément support 4 comporte un orifice traversant 40 sensiblement en son centre. D'un point de vue dimensionnel et à titre purement illustratif, l'orifice traversant 40 est sensiblement de forme rectangulaire et ses côtés mesurent une quinzaine de centimètres. Le premier élément support 4 est avantageusement métallique et plus particulièrement en acier inoxydable de sorte à éviter son oxydation au contact de l'eau et à faciliter son nettoyage. Le premier élément support 4 comporte avantageusement des poignées 41 facilitant sa manipulation. Par exemple, les poignées 41 sont des ensembles de deux orifices dans le premier élément support agencé pour permettre de saisir le premier élément support en introduisant respectivement deux doigts dans les orifices de sorte à pincer le premier élément support 4.

Le deuxième élément support 5 a par exemple sensiblement une forme de plaque. D'un point de vue dimensionnel et à titre purement illustratif, la plaque est sensiblement de forme rectangulaire et ses côtés mesurent une quinzaine de centimètres. Le deuxième élément support 5 comporte un orifice traversant 50 sensiblement en son centre. D'un point de vue dimensionnel et à titre purement illustratif, l'orifice traversant 50 est sensiblement de forme rectangulaire et ses côtés mesurent une dizaine de centimètres. Le deuxième élément support 5 est avantageusement métallique et plus particulièrement en acier inoxydable de sorte à éviter son oxydation au contact de l'eau et à faciliter son nettoyage.

Le deuxième élément support 5 est suspendu au premier élément support 4 par l'élément de suspension 6 formant liaison rotule. L'élément de suspension 6 comporte avantageusement 4 chaînes 61 reliant respectivement quatre points de l'élément support 4, à proximité des quatre coins de l'orifice 40, à quatre points de l'élément support 5, à proximité des quatre coins du support 5. Chaque chaîne 61 comporte deux extrémités, la première extrémité étant en liaison rotule avec le premier élément support 4 et la deuxième extrémité étant en liaison rotule avec le deuxième élément support 5. Selon d'autres modes de réalisation, les chaînes 61 sont remplacées par des tiges, des anneaux ou des cordes. De tels éléments de suspension 6 déformables s'étendent entre le premier élément support 4 et le deuxième élément support 5 selon une longueur par exemple d'environ 4 centimètres et permettent de maintenir le deuxième élément support sensiblement au centre de la cuve en position horizontale, l'horizontale étant définie comme étant perpendiculaire à la verticale, tout en limitant la transmission d'ondes ultrasonores à travers l'élément de suspension 6 grâce au caractère amortisseur conféré par la liaison rotule.

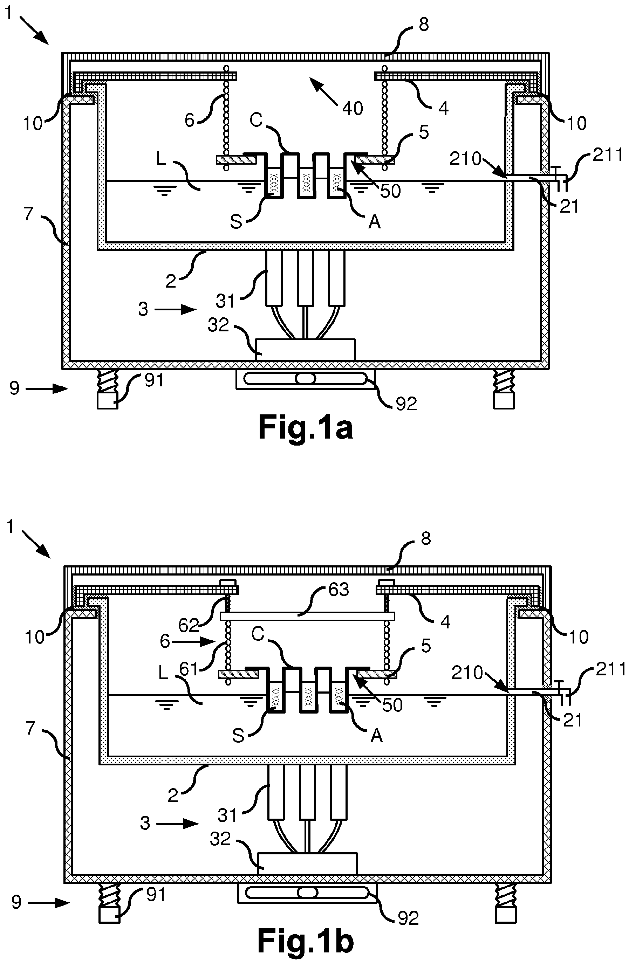

Selon un mode de réalisation particulier représenté à la figure 1b, l'élément de suspension 6 comporte un moyen de réglage agencé pour modifier la distance entre le premier élément support 4 et le deuxième élément support 5. Le moyen de réglage comporte deux traverses 63 sensiblement perpendiculaires aux chaînes 61 et quatre vis 62 reliant le premier élément support 4 aux traverses 63. En vissant ou dévissant les vis 62, la distance entre le premier élément support 4 et les traverses 63 est modifiée. Avantageusement, les quatre vis 62 sont insérées dans le premier élément support 4 respectivement en quatre points de l'élément support 4 à proximité des quatre coins de l'orifice 40. Deux vis adjacentes sont vissées respectivement dans les deux extrémités d'une première traverse, les autres vis sont vissées respectivement dans les deux extrémités de la deuxième traverse. Quatre points du deuxième élément support 5 à proximité des quatre coins de l'élément support 5 sont reliés respectivement aux quatre extrémités des deux traverses par des chaînes 61 sensiblement dans le prolongement des vis 62.

Le deuxième élément support 5 est destiné à recevoir un contenant C, typiquement une plaquette comportant une pluralité de puits pour recevoir des échantillons d'ADN. Le contenant C est placé de sorte à ce que les puits soient dans l'orifice 50 et que la direction selon laquelle s'étendent les puits soit verticale, les extrémités du contenant, en périphérie des puits, reposant sur le deuxième élément support 5.

Le deuxième élément support 5 est distant des faces latérales de la cuve d'une distance prédéterminée, par exemple une distance de 10 centimètres, de sorte à limiter l'exposition du contenant C aux effets de bord au voisinage des faces latérales créés par la réflexion des ondes ultrasonores par les faces latérales.

La cuve 2 est remplie de liquide L de sorte à immerger l'extrémité distale des puits et à laisser le deuxième élément support 5 à l'air libre afin d'éviter la réflexion des ondes ultrasonores se propageant dans le liquide L par l'élément support 5. La cuve 2 comprend par exemple entre 5 et 7 centimètres de liquide L. Lors de l'opération de remplissage de la cuve 2, le premier et le deuxième éléments support sont retirés de la cuve 2 de sorte à verser le liquide L dans la cuve 2. Le niveau de liquide L est prédéfini et doit être précis au millimètre près. Afin de contrôler précisément le niveau de liquide L dans la cuve 2, la cuve 2 comporte un dispositif de trop-plein 21. Selon le mode de réalisation représenté, le mécanisme de trop-plein comporte un orifice de vidange 210 dans une face latérale de la cuve à une hauteur correspondant au niveau de liquide souhaité. Le liquide excédentaire est évacué par un conduit 211. Le conduit 211 achemine le liquide excédentaire vers l'extérieur du châssis 7 à travers un orifice traversant dans le châssis 7. Selon un mode de réalisation non représenté, le liquide excédentaire est recueilli dans un récipient fixé au châssis 7. Selon un autre mode de réalisation non représenté, le dispositif de trop-plein 21 comporte une paroi délimitant un compartiment étanche dans la cuve 2, la paroi étant agencée pour que le liquide excédentaire passe par-dessus un bord supérieur de la paroi.

Le couvercle 8 recouvre le châssis 7 et permet d'isoler le contenu de la cuve 2 de l'environnement extérieur dans un souci de sécurité et d'hygiène. Avantageusement, le couvercle 8 comporte des joints 10 aux interfaces entre le couvercle 8 et le premier élément support 4 ou entre le couvercle 8 et la cuve 2 de sorte à limiter la propagation des ondes parasites au niveau de ces interfaces.

Le moyen de réglage de l'horizontalité 9 de la cuve comporte des pieds réglables 91 et un niveau à bulle d'air 92. Dans le mode de réalisation représenté, les pieds réglables 91 et le niveau à bulle d'air 92 sont fixés sur la face inférieure externe du châssis 7.

Le moyen de génération d'ondes ultrasonores 3 comporte une pluralité de transducteurs 31, par exemple 6 transducteurs disposés selon une grille comportant 2 lignes de 3 transducteurs afin de créer un champ d'ondes ultrasonores relativement homogène au voisinage du contenant C. Les transducteurs transforment un signal électrique en un signal mécanique générant des ondes ultrasonores. Les transducteurs sont fixés à une face externe de la cuve 2, par exemple ils sont collés au moyen d'une colle adaptée à la transmission des ondes ultrasonores ou ils sont soudés. Avantageusement, les transducteurs sont fixés sur une face inférieure externe de la cuve, à la verticale de l'orifice 50 du second élément support 5.

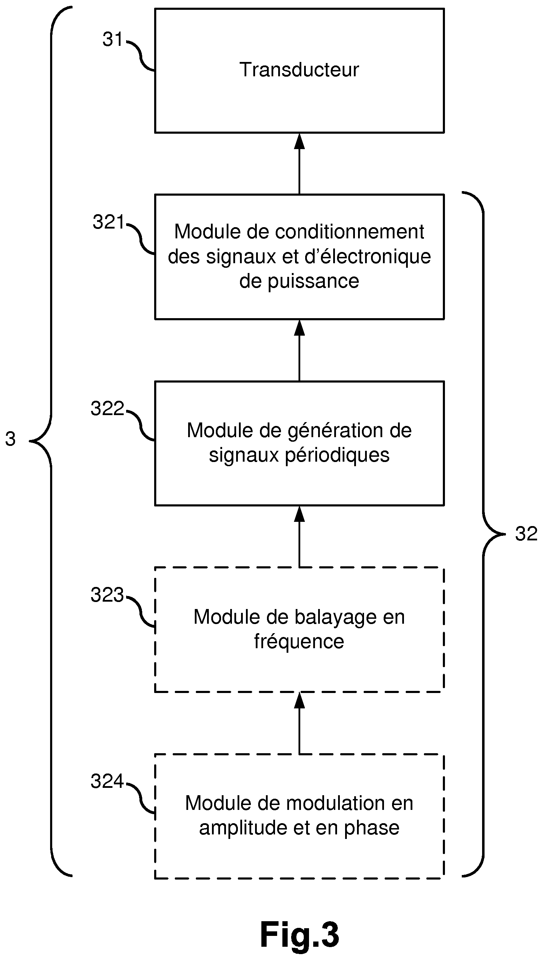

A la figure 3 est représenté un moyen de génération d'ondes ultrasonores 3. Les transducteurs 31 sont alimentés en signal électrique par un générateur de signal 32. Avantageusement, le générateur de signal 32 comporte un module de génération de signaux périodiques 322, par exemple un oscillateur piézoélectrique, et un module de conditionnement des signaux et d'électronique de puissance 321 amplifiant le signal généré par le module de génération de signaux périodiques 322. Avantageusement, afin de créer un champ d'ondes ultrasonores sensiblement homogène au voisinage du contenant et, en particulier, afin d'éviter l'apparition de ventres et de nœuds en régime stationnaire, le générateur de signal 32 comporte un module de balayage en fréquence 323 de sorte à générer au moins deux signaux ayant des fréquences distinctes dans une bande de fréquences prédéterminée, par exemple une bande de fréquence comprise entre 24kHz et 28kHz. Avantageusement, le générateur de signal 32 comporte en outre un module réglable de modulation en amplitude et en phase afin de pouvoir faire varier l'amplitude et la phase des signaux générés toujours dans l'objectif d'éviter l'apparition de ventres et de nœuds.

## Revendications

1. Dispositif (1) de segmentation d'échantillons d'ADN (A) mis en solution dans un contenant (C), le dispositif (1) comportant :
- une cuve (2) destinée à recevoir un liquide (L), la cuve (2) étant équipée d'un moyen de génération d'ondes ultrasonores (3) pour propager des ondes ultrasonores dans le liquide (L) ; et
- un premier élément support (4) reposant sur la cuve (2),
le dispositif (1) étant **caractérisé en ce qu'**il comporte en outre un deuxième élément support (5) comportant un orifice (50) traversant destiné à recevoir le contenant (C), le deuxième élément support (5) étant suspendu audit premier élément support (4) à distance dudit premier élément support (4) par au moins un élément de suspension (6) déformable formant au moins une liaison rotule de sorte à permettre l'immersion d'une portion inférieure du contenant (C) dans le liquide (L), le deuxième élément support (5) étant distant des faces latérales de la cuve (2) d'une distance prédéterminée de sorte à limiter l'exposition du contenant (C) aux effets de bord au voisinage des faces latérales créés par la réflexion des ondes ultrasonores par les faces latérales.

2. Dispositif (1) selon la revendication 1 **caractérisé en ce que** l'élément de suspension (6) comporte un moyen de réglage (62-63) agencé pour modifier la distance entre le premier élément support (4) et le deuxième élément support (5) .

3. Dispositif (1) selon l'une quelconque des revendications 1 à 2, **caractérisé en ce que** le moyen de génération d'ondes ultrasonores (3) comporte au moins un transducteur (31) ultrasonore.

4. Dispositif (1) selon la revendication 3 **caractérisé en ce que** le moyen de génération d'ondes ultrasonores (3) comporte en outre un générateur de signal (32) agencé pour alimenter en signal électrique le transducteur.

5. Dispositif (1) selon la revendication 4 **caractérisé en ce que** le générateur de signal (32) comporte un module de génération de signaux périodiques (322).

6. Dispositif (1) selon l'une quelconque des revendications précédentes **caractérisé en ce que** le moyen de génération d'ondes ultrasonores (3) est fixé sur une face inférieure externe de la cuve (2).

7. Dispositif (1) selon la revendication 6 **caractérisé en ce que** le moyen de génération d'ondes ultrasonores (3) est placé sensiblement à la verticale de l'orifice (50) du second élément support (5).

8. Dispositif (1) selon l'une quelconque des revendications précédentes **caractérisé en ce que** le premier élément support (4) comporte un orifice (40) traversant de sorte à permettre à un utilisateur d'accéder à l'orifice (50) du deuxième élément support (5).

## Patentansprüche

1. Vorrichtung (1) zur Fragmentierung von DNA-Proben (A), die in einem Behälter (C) in Lösung gebracht sind, wobei die Vorrichtung (1) Folgendes umfasst:
- ein Becken (2), das dazu bestimmt ist, eine Flüssigkeit (L) aufzunehmen, wobei das Becken (2) mit einem Mittel zum Generieren von Ultraschallwellen (3) zur Verbreitung von Ultraschallwellen in der Flüssigkeit (L) ausgerüstet ist; und
- ein erstes Stützelement (4), das auf dem Becken (2) aufliegt,
wobei die Vorrichtung (1) **dadurch gekennzeichnet ist, dass** sie ferner ein zweites Stützelement (5) umfasst, das eine durchgehende Öffnung (50) umfasst, die dazu bestimmt ist, den Behälter (C) aufzunehmen, wobei das zweite Stützelement (5) in einem Abstand zu dem ersten Stützelement (4) durch mindestens ein verformbares Aufhängungselement (6), das mindestens eine Gelenkverbindung bildet, an dem ersten Stützelement (4) aufgehängt ist, um das Eintauchen eines unteren Abschnitts des Behälters (C) in die Flüssigkeit (L) zu ermöglichen, wobei das zweite Stützelement (5) um einen vorgegebenen Abstand von den Seitenflächen des Beckens(2) entfernt ist, um die Exposition des Behälters (C) gegenüber den Randeffekten in der Nähe der Seitenflächen,
die durch die Reflexion der Ultraschallwellen durch die Seitenflächen erzeugt werden, zu begrenzen.

2. Vorrichtung (1) nach Anspruch 1, **dadurch gekennzeichnet, dass** das Aufhängungselement (6) ein Einstellmittel (62-63) umfasst, das angeordnet ist, um den Abstand zwischen dem ersten Stützelement (4) und dem zweiten Stützelement (5) zu verändern.

3. Vorrichtung (1) nach einem der Ansprüche 1 bis 2, **dadurch gekennzeichnet, dass** das Mittel zum Generieren von Ultraschallwellen (3) mindestens einen Ultraschallwandler (31) umfasst.

4. Vorrichtung (1) nach Anspruch 3, **dadurch gekennzeichnet, dass** das Mittel zum Generieren von Ultraschallwellen (3) ferner einen Signalgenerator (32) umfasst, der angeordnet ist, um den Wandler mit einem elektrischen Signal zu versorgen.

5. Vorrichtung (1) nach Anspruch 4, **dadurch gekennzeichnet, dass** der Signalgenerator (32) ein Modul zum Generieren periodischer Signale (322) umfasst.

6. Vorrichtung (1) nach einem der vorhergehenden Ansprüche, **dadurch gekennzeichnet, dass** das Mittel zum Generieren von Ultraschallwellen (3) an einer unteren Außenfläche des Beckens (2) befestigt ist.

7. Vorrichtung (1) nach Anspruch 6, **dadurch gekennzeichnet, dass** das Mittel zum Generieren von Ultraschallwellen (3) im Wesentlichen vertikal zu der Öffnung (50) des zweiten Stützelements (5) platziert ist.

8. Vorrichtung (1) nach einem der vorhergehenden Ansprüche, **dadurch gekennzeichnet, dass** das erste Stützelement (4) eine durchgehende Öffnung (40) umfasst, um einem Benutzer den Zugang zu der Öffnung (50) des zweiten Stützelements (5) zu ermöglichen.

## Claims

1. Device (1) for the fragmentation of DNA samples (A) that are in solution in a container (C), the device (1) comprising:
- a vessel (2) for receiving a liquid (L), the vessel (2) being provided with means for producing ultrasonic waves (3) so as to spread ultrasonic waves through the liquid (L); and
- a first support element (4) resting on the vessel (2),
the device (1) being **characterised in that** it further comprises a second support element (5) comprising a passage (50) designed to receive the container (C), the second support element (5) being suspended from at least one suspension element (4) at a distance from said first support element (4) by at least one deformable suspension element (6) forming at least one swivel joint in such a way as to allow for the immersion of a lower portion of the container (C) in the liquid (L), the second support element (5) is separate from the lateral faces of the vessel (2) by a predetermined distance in such a way as to limit the exposure of the container (C) to the edge effects in the vicinity of the lateral faces created by the reflection of the ultrasonic waves by the lateral faces.

2. Device (1) according to claim 1 **characterised in that** the suspension element (6) comprises a means for adjusting (62-63) arranged to modify the distance between the first support element (4) and the second support element (5).

3. Device (1) according to any of claims 1 to 2, **characterised in that** the means for producing ultrasonic waves (3) comprises at least one ultrasonic transducer (31).

4. Device (1) according to claim 3 **characterised in that** the means for producing ultrasonic waves (3) further comprises a signal generator (32) arranged to supply the transducer with an electric signal.

5. Device (1) according to claim 4 **characterised in that** the signal generator (32) comprises a module for generating periodic signals (322).

6. Device (1) according to any preceding claim **characterised in that** the means for producing ultrasonic waves (3) is fastened onto an outer lower face of the vessel (2) .

7. Device (1) according to claim 6 **characterised in that** the means for producing ultrasonic waves (3) is placed substantially vertically with the passage (50) of the second support element (5).

8. Device (1) according to any preceding claim **characterised in that** the first support element (4) comprises a passage (40) in such a way as to allow a user to access the passage (50) of the second support element (5).
